# EUROPEAN PATENT APPLICATION

(11) **EP 4 567 108 A1**
(43) Date of publication of application: **11.06.2025**
(21) Application number: 23214288.5
(22) Date of filing: 05.12.2023
(51) Int. Cl.: C12N 15/10, C12N 15/62, C40B 40/10

(54) **SCREENING OF TARGET PEPTIDES USING FUSION POLYPEPTIDES**

(71) Applicant: Kutzner, Christoph, 68219 Mannheim (DE); Stähler, Peer, 68167 Mannheim (DE)
(72) Inventor: Kutzner, Christoph, 68219 Mannheim (DE); Stähler, Peer, 68167 Mannheim (DE)
(74) Representative: Eisenführ Speiser

(57) **Abstract**

The present invention lies in the field of peptide screening and provides methods for the targeted screening of peptides of interest. Furthermore, the present invention provides tagged fusion polypeptides carrying the target peptide to be screened as well as peptide libraries for screening target peptides. The invention additionally provides kits for screening target peptides.

## Description

The present invention lies in the field of peptide screening and provides methods for the targeted screening of peptides of interest. Furthermore, the present invention provides tagged fusion polypeptides carrying the target peptide to be screened as well as peptide libraries for screening target peptides. The invention additionally provides kits for screening target peptides.

Since late 1990, there have been several advances in preparing and screening large numbers of various peptides. Developments have continued in methods of peptide screening based on peptides exposition on coat proteins, produced via fusion coliphage constructs. Further developments have been made in increasing the multitude of peptides produced by chemical synthesis. The chemical synthesis has the drawback that it is a costly and time-consuming process and not all desired peptides can be produced at an industrial scale. For example, peptides with a high content of hydrophobic amino acids are not producible by chemical synthesis and it is especially challenging to produce peptides with an N-terminal proline. Furthermore, the chemical synthesis requires the use of harsh reagents and costly purification steps.

The screening of peptides is a valuable analytical tool to determine e.g. the biochemical and/or pharmacological properties of peptides. For this, a sufficient amount of peptide needs to be produced, which is then used in the assay to be performed, e.g. for discovering new active pharmaceutical ingredients.

WO 2012219585 A1 teaches a method for producing target peptides in large scale using fusion polypeptides. In the disclosed methods, peptides can be produced in a high amount and at decreased costs due to the straightforward and large-scale suitable purification process. This technique enables the production of a sufficient amount of peptides to be used in screening assays at moderate costs. However, for each screening assay, a large variety of peptides needs to be produced. This means that one peptide is produced per production cycle.

Thus, the primary task of the present invention was to provide a screening method, which enables the screening of a variety of peptides in a short time and at decreased costs.

This primary task of the invention was solved by providing a method for screening target peptides, comprising or consisting of the steps
a) Establishing a peptide library comprising at least two plasmids carrying the genetic information of a fusion polypeptide comprising in N- to C-terminal direction: (i) an inclusion body targeting sequence, (ii) a binding domain, (iii) an autoprotease domain, and (iv) a target peptide to be screened;
b) Transforming the peptide library into at least one organism for expressing the fusion polypeptide carrying the target peptide;
c) Cultivating the at least one transformed organism under conditions allowing the expression of the fusion polypeptide carrying the target peptide and its deposition in inclusion bodies;
d) Obtaining at least two different inclusion bodies, each including one species of tagged fusion polypeptide, wherein the tagged fusion polypeptide comprises or consists of the fusion polypeptide carrying the target peptide and the plasmid or a fragment thereof, which carries the genetic information for expressing the respective fusion polypeptide, bound to the binding domain of the fusion polypeptide;
e) Physically separating and purifying the inclusion bodies;
f) Refolding of the fusion polypeptide from the separated inclusion bodies;
g) Activating the autoprotease domain and separating the target peptide from the fusion polypeptide, thereby obtaining a mixture of target peptide and remaining fusion polypeptide;
h) Performing assays with the obtained target peptide in step g),
wherein the target peptide is identified in step g) or h) by extracting the remaining fusion polypeptide from the mixture obtained in step g) and identifying the plasmid completely or partly bound to the binding domain of the remaining fusion polypeptide.

Step a) of establishing a peptide library preferably includes the generation of plasmids carrying the genetic information of a peptide, which should be used in a screening assay. For this, a nucleic acid sequence encoding the peptide of interest is synthesized and cloned into a plasmid, wherein the plasmid comprises the genetic information for the fusion polypeptide having in inclusion body targeting sequence (i), a binding domain (ii) and an autoprotease domain (iii). The binding domain (ii) is able to bind nucleic or ribonucleic acids. The nucleic acid encoding the target peptide is then cloned into the plasmid in such a way that it directly follows the nucleic acid sequence of the autoprotease. Methods for cloning of nucleic acid sequences in plasmid are known to the person skilled in the art.

The peptide library comprises at least two different plasmids, wherein the plasmids preferably differ in their target peptide sequence. Preferably, the peptide library comprises at least three, at least four, at least five, at least ten, at least 20, at least 50 different target peptides. It is also preferred in terms of the present invention that the peptide library comprises more than 50 different target peptides.

In the subsequent method step b), the established peptide library of a) is transformed into a suitable host cell. "Transforming" in terms of the present invention also means "transfecting". Suitable methods for transforming a plasmid into a host cell are known to the person skilled in the art and all involve bringing the plasmid carrying the genetic information for the fusion polypeptide into a suitable host cell, in which the fusion polypeptide with the target peptide is expressed.

For expression, the transformed organism or host cell needs to be cultivated under conditions, which are suitable for expressing the fusion polypeptide and target peptide.

Such conditions are dependent on the host cell and are known to the person skilled in the art.

As the fusion polypeptide comprises an inclusion body targeting sequence, the fusion polypeptide with the target peptide is deposited in inclusion bodies. It was surprisingly found in terms of the present invention that each inclusion body only comprises one species of fusion polypeptide with the target peptide, even though the cultivated host cell(s) comprise the variety of the peptide library. Thus, it was found that each inclusion body only comprises one variety of peptides. Furthermore it was found that in the inclusion bodies, the fusion polypeptides comprise the plasmid encoding the genetic information of the target peptide. The plasmid is bound to the binding domain as this domain is able to bin nucleic acids. Thus, in step d) of the present invention, a tagged polypeptide is obtained carrying the genetic information of the target peptide.

In step e) of the method according to the invention, the inclusion bodies are physically separated and step f) includes the refolding of the fusion polypeptide from the inclusion bodies to yield its active confirmation.

Preferably after physically separating the inclusion bodies, they are preferably subjected to reaction tubes, which are then used in the screening assay to be performed. Moreover preferably, the inclusion bodies are physically separated and subjected to a multi-well plate, wherein each well comprises one inclusion body. The subsequent steps are then performed in the multi-well plate. The method according to the present invention can thus be used together with high throughput screening methods.

After refolding of the fusion polypeptides from the inclusion bodies, the autoprotease of the fusion polypeptide is activated, thereby releasing the target peptide from the fusion polypeptide (step g)). This results in the products target peptide and the remaining fusion polypeptides with the bound (tagged) plasmid. The remaining target plasmid can then be identified for the target peptide of this fusion polypeptide. Suitable identification methods involve preferably sequencing of the plasmid or by screening PCR targeting the fusion polypeptide and/or target peptide (the amplified sequence is then subjected to sequencing).

In the next step h), the screening assay is performed with the target peptide as obtained in step g). Such screening assay may include e.g. any bioactivity assay or binding assay that can be assessed in a plate reader using fluorescence, absorption or fluorescence anisotropy as a read-out. Alternatively, the bioactivity of antimicrobial / antifungal / antialgae peptides can be tested using solid agar or liquid LB colony forming unit tests. In this step, the target peptide is identified by a method as described above. For this, the plasmid bound to the fusion polypeptide is analyzed and the target peptide can be identified.

"Extracting the fusion polypeptide" in terms of the present invention means the (partial) removal of the fusion polypeptide from the reaction mixture, which is present in e.g. a reaction tube or a multi-well plate. This can be done by standard operations, such as taking a sample from e.g. the reaction tube or multi-well plate and performing the identification on the acquired sample.

Whenever the present disclosure relates to the percentage of identity of nucleic acid or amino acid sequences to each other these values define those values as obtained by using the EMBOSS Water Pairwise Sequence Alignments (nucleotide) program or the EMBOSS Water Pairwise Sequence Alignments (protein) program for amino acid sequences. Alignments or sequence comparisons as used herein refer to an alignment over the whole length of two sequences compared to each other. Those tools provided by the European Molecular Biology Laboratory (EMBL) European Bioinformatics Institute (EBI) for local sequence alignments use a modified Smith-Waterman algorithm (see Smith, T.F. & Waterman, M.S. "identification of common molecular subsequences" Journal of Molecular Biology, 1981 147 (1):195-197). When conducting an alignment, the default parameters defined by the EMBL-EBI are used. Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5. The skilled person is well aware of the fact that, for example, a sequence encoding a polypeptide can be "codon-optimized" if the respective sequence is to be used in another organism in comparison to the original organism a molecule originates from.

It is preferred in terms of the method for screening peptides according to the present invention that the binding domain (ii) of the fusion polypeptide comprises or consists of an amino acid according to SEQ ID NO.: 1 or an amino acid sequence having at least 90 %, 91 %, 92 %, 93 %, 94 %, 95 %, 96 %, 97 %, 98 % or 99 % sequence identity to an amino acid sequence according to SEQ ID NO.: 1.

The binding domain fulfils the function that the plasmid encoding the fusion polypeptide and the target peptide binds to the binding domain and thus functions as a tag for analysing, which target peptide is present in the corresponding inclusion body.

It is furthermore preferred in terms of the method according to the invention that the autoprotease domain (iii) of the fusion polypeptide comprises or consists of an amino acid sequence according to SEQ ID NO.: 2 or 3 or an amino acid sequence having at least 90 %, 91 %, 92 %, 93 %, 94 %, 95 %, 96 %, 97 %, 98 % or 99 % sequence identity to an amino acid sequence selected according to SEQ ID NO.: 2 or 3.

The autoprotease domain fulfils the function to release the target peptide from the fusion polypeptide at suitable conditions as described herein.

Moreover it is preferred in terms of the method according to the invention that the inclusion body targeting sequence (i) of the fusion polypeptide comprises or consists of an amino acid sequence according to SEQ ID NO.: 4 or an amino acid sequence having at least 90 %, 91 %, 92 %, 93 %, 94 %, 95 %, 96 %, 97 %, 98 % or 99 % sequence identity to an amino acid sequence selected from SEQ ID NO.: 4.

It is also preferred that the fusion polypeptide additionally comprises a linker sequence (v) between the binding domain (ii) and the autoprotease domain (iii). Preferably, the linker sequence (v) comprises an N-terminal alpha helix and/or a C-terminal sequence of a random coil structure. Especially preferably, the linker sequence (v) comprises or consists of an amino acid sequence according to SEQ ID NO.: 5 or an amino acid sequence having at least 90 %, 91 %, 92 %, 93 %, 94 %, 95 %, 96 %, 97 %, 98 % or 99 % sequence identity to an amino acid sequence according to SEQ ID NO.: 5.

It is also preferred in terms of the method according to the invention that the fusion polypeptide comprises or consists of an amino acid sequence selected from SEQ ID NO.: 6 or 7 or an amino acid sequence having at least 90 %, 91 %, 92 %, 93 %, 94 %, 95 %, 96 %, 97 %, 98 % or 99 % sequence identity to an amino acid sequence selected from SEQ ID NO.: 6 or 7.

It is preferred that the organism used in step b) and c) of the present invention is selected from *Escherichia coli, Vibrio natrigens, Saccheromyces cerevisiae, Aspergillus niger,* green algae, microalgae, HEK T293 and Chinese hamster ovary cells (CHO). Conditions suitable for the cultivation of these organisms are known to the person skilled in the art.

In terms of the present invention, the term organism, expression organism and host cell have the same meaning.

It is furthermore preferred in terms of the method according to the invention that the step of physically separating the inclusion bodies is done using a method selected from the group consisting of flow cytometry, gel filtration, manual dilution and size exclusion chromatography.

Moreover, it is preferred in terms of the method according to invention that the obtained inclusion bodies in step d) each comprise an amount of more than 250.000 copies of the tagged fusion polypeptide, preferably more than 400.000 and especially preferably more than 500.000 copies.

Furthermore, it is preferred in terms of the present invention that the refolding in step f) is done under neutral or mildly basic conditions. Preferably, the refolding takes place in a buffer comprising 6 M guanidinium chloride, 8 M urea or 2 % sodium dodecyl sulfate.

It is also preferred in terms of the present invention that the activation of the fusion polypeptide in step g) is done at a pH value of between 6.8 and 7.2.

Another aspect of the present invention relates to a tagged fusion polypeptide, comprising or consisting of a fusion polypeptide as defined in claims 1 to 5 carrying a target peptide and a plasmid encoding the fusion polypeptide carrying the target peptide bound to the binding domain of the fusion polypeptide.

The complex of plasmid bound to the fusion polypeptide is also called a bioportid.

Yet another aspect of the present invention relates to a peptide library for screening peptides comprising or consisting of at least two different plasmids carrying the genetic information for expressing a fusion polypeptide comprising in N- to C-terminal direction: (i) an inclusion body targeting sequence, (ii) a binding domain, (iii) an autoprotease domain, and (iv) the target peptide to be screened. Preferably, the plasmid used is a pET vector, which is well known in the art.

It is preferred that the peptide library according to the invention comprises at least ten, preferably at least 20, especially preferably at least 50, different plasmids carrying the genetic information for expressing a fusion polypeptide as described herein. This means that the peptide library comprises the genetic information for at least 20, especially preferably at least 50, different target peptides. Preferably, the peptide library comprises more than 50 different target peptides.

Another aspect of the present invention relates to a kit for screening peptides comprising:
- at least one plasmid carrying the genetic information of a fusion polypeptide comprising in N- to C-terminal direction: (i) an inclusion body targeting sequence (ii) a binding domain, (iii) an autoprotease domain, wherein the peptide to be screened is inserted after the autoprotease domain,
- means for transforming a peptide to be screened into the plasmid.

It is preferred that the binding domain (ii) of the fusion polypeptide comprises or consists of an amino acid sequence according to SEQ ID NO.:1 or an amino acid sequence having at least 90 %, 91 %, 92 %, 93 %, 94 %, 95 %, 96 %, 97 %, 98 % or 99 % sequence identity to an amino acid sequence according to SEQ ID NO.: 1.

Preferably, the autoprotease domain (iii) of the fusion polypeptide comprises or consists of an amino acid sequence selected from SEQ ID NO.: 2 or 3 or an amino acid sequence having at least 90 %, 91 %, 92 %, 93 %, 94 %, 95 %, 96 %, 97 %, 98 % or 99 % sequence identity to an amino acid sequence selected from SEQ ID NO.: 2 or 3.

Also preferably, the inclusion body targeting sequence (i) of the fusion polypeptide comprises or consists of an amino acid sequence according to SEQ ID NO.: 4 or an amino acid sequence having at least 90 %, 91 %, 92 %, 93 %, 94 %, 95 %, 96 %, 97 %, 98 % or 99 % sequence identity to an amino acid sequence according to SEQ ID NO.: 4.

Moreover preferably, the fusion polypeptide additionally comprises a linker sequence (v) between the binding domain (i) and the autoprotease domain (ii), preferably, wherein the linker sequence (v) comprises an N-terminal alpha helix and/or a C-terminal sequence of a random coil structure, especially preferably wherein the linker sequence (v) comprises or consists of an amino acid sequence according to SEQ ID NO.: 5 or an amino acid sequence having at least 90 %, 91 %, 92 %, 93 %, 94 %, 95 %, 96 %, 97 %, 98 % or 99 % sequence identity to an amino acid sequence according to SEQ ID NO.:5.

### Short description of sequences

**SEQ ID NO.: 1** is the amino acid sequence encoding the binding domain.
**SEQ ID NO.: 2 and 3** are amino acid sequences encoding autoprotease domains.
**SEQ ID NO.: 4** is the amino acid sequence encoding the inclusion body sequence.
**SEQ ID NO.: 5** is the amino acid sequence encoding the linker sequence.
**SEQ ID NO.: 6 and 7** are amino acid sequences of fusion polypeptides to be used according to the invention.
**SEQ ID NO.: 8** is the nucleic acid sequence encoding the binding domain.
**SEQ ID NO.: 9 and 10** are the nucleic acid sequences encoding autoprotease domains.
**SEQ ID NO.: 11** is the nucleic acid sequence encoding the inclusion body sequence.
**SEQ ID NO.: 12** is the nucleic acid sequence encoding the linker.
**SEQ ID NO.: 13 and 14** are nucleic acid sequences of fusion polypeptides to be used according to the invention.

### Examples

### Example 1

Derivatives of the peptide melittin were each cloned at the C-terminus of the fusion polypeptide according to SEQ ID NO.: 6 into a pET vector (Merck Millipore, Darmstadt, Germany). A standard cloning protocol using the restriction sites Ncol or Ndel and EcoRI of any vector of the pET family to insert the fusion polypeptide gene is used.

5 µl of plasmid solution comprising a DNA concentration of 100 ng/µl are transferred into a 2ml reaction vessel containing 50µl of pre thawed E. coli BL21 pLys or any other BL21 derivative and transformed via heat shock.

The obtained cells were then cultivated. The expression culture is grown in a 2 I shaking bottle and induced with a suitable amount of a chemical inducer, including lactose, rhamnose and Isopropyl-β-D-thiogalactopyranosid (IPTG). The culture is grown at a temperature of 37°C, agitated at shaking between 150 and 250 rpm to the exponential growth phase, and induced. The expression culture is run for six hours and discretely supplemented with glucose or glycerol (both 20 g/I), thiamine, citric acid and a suitable trace element solution. The solutions are added at a rate of 0.1 ml/min for 3.5 hour and an additional hour at 0.2 ml/min. The culture is harvested by centrifugation at 4°C and 3000 rpm for 20 minutes and the cells are subjected to cell lysis.

The obtained cells were lysed in a lysis buffer (0 mM and 75 mM sodium acetate, 0 mM to 20 mM 2-[4-(2-hydroxyethyl)piperazin-1-yl]ethane sulfonic acid (HEPES), 2 mM Magnesium chloride and 1 % Triton X-100, pH > 9.0). Lysis can be performed using sonication, dispersion or French press. The obtained inclusion bodies are separated by gel filtration and each inclusion body is then transferred into one well of a 96 well plate (Merck, Darmstadt, Germany). Refolding of the fusion polypeptide from the inclusion bodies is performed as described herein. One row of the multi-well plate is reserved for the positive control including wells with purified melittin. Thus, the activity of the derivatives can afterwards determined based on the activity of unmodified mellitin.

The antimicrobial activity of melittin and its derivatives was tested with a cytotoxicity assay as described in Askari et al. "In vitro and in vivo toxicity and antibacterial efficacy of melittin against clinical extensively drug-resistant bacteria." BMC Pharmacol Toxicol 22, 42 (2021).

In a next step, a screening PCR is performed in each well of the assay to identify the genetic sequence of the peptide within the well. The peptides with the highest cytotoxicity in relation to the purified melittin were selected for further evaluation.

## Claims

1. Method for screening target peptides, comprising or consisting of the steps
a) Establishing a peptide library comprising at least two plasmids carrying the genetic information of a fusion polypeptide comprising in N- to C-terminal direction: (i) an inclusion body targeting sequence, (ii) a binding domain, (iii) an autoprotease domain, and (iv) a target peptide to be screened;
b) Transforming the peptide library into at least one organism for expressing the fusion polypeptide carrying the target peptide;
c) Cultivating the at least one transformed organism under conditions allowing the expression of the fusion polypeptide carrying the target peptide and its deposition in inclusion bodies;
d) Obtaining at least two different inclusion bodies, each including one species of tagged fusion polypeptide, wherein the tagged fusion polypeptide comprises or consists of the fusion polypeptide carrying the target peptide and the plasmid or a fragment thereof, which carries the genetic information for expressing the respective fusion polypeptide, bound to the binding domain of the fusion polypeptide;
e) Physically separating and purifying the inclusion bodies;
f) Refolding of the fusion polypeptide from the separated inclusion bodies;
g) Activating the autoprotease domain and separating the target peptide from the fusion polypeptide, thereby obtaining a mixture of target peptide and remaining fusion polypeptide;
h) Performing assays with the obtained target peptide in step g),
wherein the target peptide is identified in step g) or h) by extracting the remaining fusion polypeptide from the mixture obtained in step g) and identifying the plasmid completely or partly bound to the binding domain of the remaining fusion polypeptide.

2. Method for screening peptides according to claim 1, wherein the binding domain (ii) of the fusion polypeptide comprises or consists of an amino acid according to SEQ ID NO.: 1 or an amino acid sequence having at least 90 %, 91 %, 92 %, 93 %, 94 %, 95 %, 96 %, 97 %, 98 % or 99 % sequence identity to an amino acid sequence according to SEQ ID NO.: 1.

3. Method for screening peptides according to claim 1 or 2, wherein the autoprotease domain (iii) of the fusion polypeptide comprises or consists of an amino acid sequence according to SEQ ID NO.: 2 or 3 or an amino acid sequence having at least 90 %, 91 %, 92 %, 93 %, 94 %, 95 %, 96 %, 97 %, 98 % or 99 % sequence identity to an amino acid sequence selected according to SEQ ID NO.: 2 or 3.

4. Method for screening peptides according to any one of the previous claims, wherein the inclusion body targeting sequence (i) of the fusion polypeptide comprises or consists of an amino acid sequence according to SEQ ID NO.: 4 or an amino acid sequence having at least 90 %, 91 %, 92 %, 93 %, 94 %, 95 %, 96 %, 97 %, 98 % or 99 % sequence identity to an amino acid sequence selected from SEQ ID NO.: 4, and/or
wherein the fusion polypeptide additionally comprises a linker sequence (v) between the binding domain (ii) and the autoprotease domain (iii), preferably, wherein the linker sequence (v) comprises an N-terminal alpha helix and/or a C-terminal sequence of a random coil structure, especially preferably wherein the linker sequence (v) comprises or consists of an amino acid sequence according to SEQ ID NO.: 5 or an amino acid sequence having at least 90 %, 91 %, 92 %, 93 %, 94 %, 95 %, 96 %, 97 %, 98 % or 99 % sequence identity to an amino acid sequence according to SEQ ID NO.: 5.

5. Method for screening peptides according to any one of the previous claims, wherein the fusion polypeptide comprises or consists of an amino acid sequence selected from SEQ ID NO.: 6 or 7 or an amino acid sequence having at least 90 %, 91 %, 92 %, 93 %, 94 %, 95 %, 96 %, 97 %, 98 % or 99 % sequence identity to an amino acid sequence selected from SEQ ID NO.: 6 or 7.

6. Method for screening peptides according to any one of the previous claims, wherein the expression organism is selected from *Escherichia coli, Vibrio natrigens, Saccheromyces cerevisiae, Aspergillus niger,* green algae, microalgae, HEK T293 and Chinese hamster ovary cells (CHO).

7. Method for screening peptides according to any one of the previous claims, wherein the step of physically separating the inclusion bodies is done using a method selected from the group consisting of flow cytometry, gel filtration, manual dilution and size exclusion chromatography.

8. Method for screening peptides according to any one of the previous claims, wherein the obtained inclusion bodies in step d) each comprise an amount of more than 250.000 copies of the tagged fusion polypeptide, preferably more than 400.000 and especially preferably more than 500.000 copies.

9. Method for screening peptides according to any one of the previous claims, wherein the refolding in step f) is done under neutral or mildly basic conditions.

10. Method for screening peptides according to any one of the previous claims, wherein the activation of the fusion polypeptide in step g) is done at a pH value of between 6.8 and 7.2.

11. Tagged fusion polypeptide, comprising or consisting of a fusion polypeptide as defined in claims 1 to 5 carrying a target peptide and a plasmid encoding the fusion polypeptide carrying the target peptide bound to the binding domain (ii) of the fusion polypeptide.

12. Peptide library for screening peptides comprising or consisting of at least two different plasmids carrying the genetic information for expressing a fusion polypeptide comprising in N- to C-terminal direction: (i) an inclusion body targeting sequence, a binding domain, (ii), an autoprotease domain (iii), and (iv) the target peptide to be screened.

13. Peptide library according to claim 12, wherein the peptide library comprises at least ten, preferably at least 20, especially preferably at least 50, different plasmids carrying the genetic information for expressing a fusion polypeptide as described in claim 12.

14. Kit for screening peptides comprising:
- at least one plasmid carrying the genetic information of a fusion polypeptide comprising in N- to C-terminal direction: (i) an inclusion body targeting sequence (i), a binding domain (ii), an autoprotease domain(iii), and the target peptide to be screened (iv), wherein the peptide to be screened is inserted after the inclusion body targeting sequence,
- means for transforming a peptide to be screened into the plasmid.

15. Kit for screening peptides according to claim 14, wherein the binding domain (ii) of the fusion polypeptide comprises or consists of an amino acid sequence according to SEQ ID NO.:1 or an amino acid sequence having at least 90 %, 91 %, 92 %, 93 %, 94 %, 95 %, 96 %, 97 %, 98 % or 99 % sequence identity to an amino acid sequence according to SEQ ID NO.: 1,
and/or
wherein the autoprotease domain (iii) of the fusion polypeptide comprises or consists of an amino acid sequence selected from SEQ ID NO.: 2 or 3 or an amino acid sequence having at least 90 %, 91 %, 92 %, 93 %, 94 %, 95 %, 96 %, 97 %, 98 % or 99 % sequence identity to an amino acid sequence selected from SEQ ID NO.: 2 or 3,
and/or
wherein the inclusion body targeting sequence (i) of the fusion polypeptide comprises or consists of an amino acid sequence according to SEQ ID NO.: 4 or an amino acid sequence having at least 90 %, 91 %, 92 %, 93 %, 94 %, 95 %, 96 %, 97 %, 98 % or 99 % sequence identity to an amino acid sequence according to SEQ ID NO.: 4,
and/or
wherein the fusion polypeptide additionally comprises a linker sequence (v) between the binding domain (ii) and the autoprotease domain (iii), preferably, wherein the linker sequence (v) comprises an N-terminal alpha helix and/or a C-terminal sequence of a random coil structure, especially preferably wherein the linker sequence comprises or consists of an amino acid sequence according to SEQ ID NO.: 5 or an amino acid sequence having at least 90 %, 91 %, 92 %, 93 %, 94 %, 95 %, 96 %, 97 %, 98 % or 99 % sequence identity to an amino acid sequence according to SEQ ID NO.:5.
